(19) Europäisches Patentamt
European Patent Office
Office européen des brevets

(11) **EP 2 882 585 B1**

## (12) EUROPEAN PATENT SPECIFICATION

(45) Date of publication and mention
of the grant of the patent:
**31.03.2021 Bulletin 2021/13**

(51) Int Cl.:
**B32B 5/02** (2006.01)    **B32B 5/26** (2006.01)
**A61F 13/514** (2006.01)    **A61F 13/513** (2006.01)
**A61F 13/511** (2006.01)    **A61F 13/15** (2006.01)

(21) Application number: **13753373.3**

(22) Date of filing: **12.08.2013**

(86) International application number:
**PCT/US2013/054477**

(87) International publication number:
**WO 2014/028362 (20.02.2014 Gazette 2014/08)**

(54) **MULTILAYERED NONWOVEN WEBS WITH VISUALLY DISTINCT BOND SITES AND METHOD OF MAKING**

MEHRSCHICHTIGE VLIESE MIT VISUELL UNTERSCHIEDLICHEN BINDUNGSSTELLEN UND HERSTELLUNGSVERFAHREN DAFÜR

BANDES NON TISSÉES À COUCHES MULTIPLES PRÉSENTANT DES RÉGIONS DE COLLAGE VISUELLEMENT DISTINCTES ET PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2012 US 201261682480 P**

(43) Date of publication of application:
**17.06.2015 Bulletin 2015/25**

(73) Proprietor: **The Procter & Gamble Company
Cincinnati, OH 45202 (US)**

(72) Inventors:
• **BILLS, Michael**
  **Mason, Ohio 45040 (US)**
• **BUSAM, Ludwig**
  **65510 Huenstetten (DE)**
• **DE BEER, Antonius, Lambertus**
  **Loveland, Ohio 45140 (US)**
• **ENDRES, Joerg**
  **60388 Frankfurt (DE)**
• **HERRON, Carlisle, Mitchell**
  **Cincinnati, Ohio 45236 (US)**
• **ISELE, Olaf, Erik Alexander**
  **West Chester, Ohio 45069 (US)**
• **PURDON, Mike**
  **Hebron, Kentucky 41048 (US)**
• **SAEVECKE, Dirk**
  **65185 Wiesbaden (DE)**
• **XU, Han**
  **Cincinnati, Ohio 45236 (US)**

(74) Representative: **P&G Patent Germany
Procter & Gamble Service GmbH
Sulzbacher Straße 40
65824 Schwalbach am Taunus (DE)**

(56) References cited:
WO-A1-00/38915    WO-A1-2005/095700
WO-A1-2011/025486    WO-A2-2006/056892
US-A1- 2011 104 459    US-B2- 6 557 224

**Description**

BACKGROUND OF THE INVENTION

Nonwoven webs are widely used in disposable absorbent articles for personal care and hygiene, such as disposable diapers, training pants, adult incontinence undergarments, feminine hygiene products, breast pads, care mats, bibs, wound dressing products, and the like. Also in disposable cleaning articles, such as sweeper or mops, nonwoven webs find intensive application. To make these disposable absorbent articles and disposable cleaning articles more appealing to the consumers, the nonwoven webs used therein are often colored or provided with a printed pattern or graphic. Apart from increasing the overall visual appearance of the disposable absorbent articles and disposable cleaning articles, it is often desirable to provide signals to the consumer to highlight certain aspects of features of the disposable absorbent articles, such as softness of the inner and outer surfaces of the articles, or the ability to absorb liquid.

Uniformly colored nonwoven webs (e.g., by using colored fibers) pose certain restrictions on the ability to accentuate specific aspects and features as distinct areas within a given nonwoven web cannot be visually set apart from the remaining nonwoven web.

On the other side, printing images on nonwoven webs results in an increase of cost. It requires an additional process step, namely the printing step, in addition to the manufacturing of the nonwoven web. Also, e.g. when used in disposable absorbent articles, the printed images may be rubbed off during use, e.g. when the print is provided on a surface of a nonwoven web which forms a garment-facing surface of the article. Also, if the print is applied on the nonwoven web which forms the inner surface of a disposable absorbent article (such as the topsheet), the inks have to be compatible with surfactants and/or the lotion with which the topsheet may have been treated and must not be washed off when they come into contact with body liquids.

Hence, there is a continued need to provide nonwoven webs having a visual distinct appearance, which can be made in a simple, cost-efficient manner and which do not cause drawbacks, such as rub-off, wash off, or adverse effects on additional treatments of the nonwoven webs, such as application of lotion and/or surfactant.

WO2006056892 teaches a method of forming a multi-colored multi-layered non-woven composite includes the application of ultrasonic energy to the top layer to displace a portion of that layer in a predetermined pattern such that openings are formed through which any underlying layer may be visible. In related embodiments, the use of pinsonic or ultrasonic embossing of a non-woven layer provides a three-dimensional colored pattern due to melting and densification of the fibers and/or the activation of heat activatable pigments or dyes used to color the non-woven.

US6557224 relates to a colored patterning of a web-shaped nonwoven or a composite made of a nonwoven and a fabric or knit is achieved by water jet needling. The nonwoven provided as the upper layer of two layers is provided with one or more colors or is colored or printed itself and is placed on a second nonwoven or a woven or one that has a different color. Then both layers are subjected to the water jets that displace the fibers, with the colored fibers in the first layer being displaced into the second layer to produce a pattern on the underside of the second layer. It is also possible, instead of colored fibers in the nonwoven of the upper layer, to move them when they are not colored into a second layer that can have any color.

SUMMARY OF THE INVENTION

The invention refers to a multilayered nonwoven web comprising at least a first and a second layer, the first layer forming the first outer surface of the multilayered nonwoven web. The fibers of the first layer and the fibers of the second layer differ from each other in color. The multilayered nonwoven web is pattern bonded, wherein the bonded areas on the first outer surface have a first color and the unbonded areas on the first outer surface have a second color which is different from the first color. When viewed from the first outer surface, the delta E* between the bonded areas and the unbonded areas is at least 0.7, or at least 1.0, or at least 2.5, or at least 3.0, or at least 4.0, or at least 5.0, or at least 10 or at least 15.

The bonded areas may be provided by use of heat, pressure, ultrasonic or any combination thereof.

The invention further discloses a method of making a multilayered nonwoven web, the method comprising the steps of

- laying down a first fibrous layer; and

- laying down a second fibrous layer, wherein the fibers of the first layer and fibers of the second layer differ from each other in color;

- bonding the first and second layers to each other by a bond pattern,

wherein the bonded areas on the first surface have a first color and the unbonded areas on the first surface have a second color, which is different from the first color and wherein on the first outer surface, the delta E* between the bonded areas and the unbonded areas is at least 0.7, or at least 1.0, or at least 2.5, or at least 3.0, or at least 4.0, or at least 5.0, or at least 10 or at least 15.

The multilayered nonwoven web may have a bond pattern which is a consumer noticeable pattern, especially if the multilayered nonwoven web is comprised by a disposable absorbent article, such as a disposable diaper.

For the avoidance of doubt, the terms "first layer" and "second layer" as used herein do not reflect the order in which the layers are laid down and assembled during manufacture, i.e. the first layer may be laid down before the second layer or vice versa.

As the color difference between the bonded areas and the unbonded areas is due to the color difference of the fibers comprised by the different layers of the multilayered nonwoven web (hence, not due to a print provided on a surface of the multilayered nonwoven web), possible rub-off of color during use is largely reduced. The multilayered nonwoven web may have a color fastness rating of 3.5 and above, or 4 and above. Such color fastness ratings reflect insignificant or no ruff-off of color.

## BRIEF DESCRIPTION OF THE DRAWINGS

These and other features, aspects and advantages of the present invention will become better understood with regard to the following description, appended claims, and accompanying drawing where:

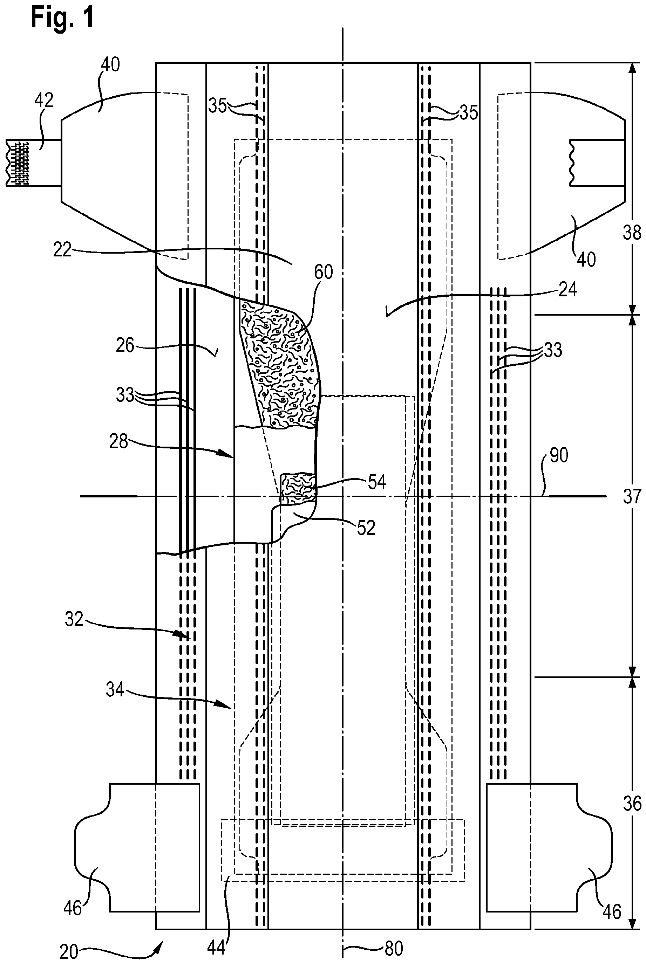

Fig. 1 is a plan view of a diaper as an exemplary disposable absorbent article which may comprise the multilayered nonwoven web of the present invention.

## DETAILED DESCRIPTION OF THE INVENTION

Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers (baby diapers or diapers for adult incontinence), pants, feminine hygiene products such as sanitary napkins or sanitary pads, breast pads, care mats, bibs, wipes, and the like. As used herein, the term "exudates" includes, but is not limited to, urine, blood, vaginal discharges, breast milk, sweat and fecal matter. Preferred absorbent articles of the present invention are diapers, pants, sanitary napkins, sanitary pads and wipes, such as wet wipes for personal hygiene use.

"Bicomponent" refers to fibers having a cross-section comprising two discrete polymer components, two discrete blends of polymer components, or one discrete polymer component and one discrete blend of polymer components. "Bicomponent fiber" is encompassed within the term "Multicomponent fiber." A Bicomponent fiber may have an overall cross section divided into two subsections of the differing components of any shape or arrangement, including, for example, concentric core-and-sheath subsections, eccentric core-and-sheath subsections, side-by-side subsections, radial subsections, etc.

"Bond Area Percentage" on a nonwoven web is a ratio of area occupied by bond impressions, to the total surface area of the web, expressed as a percentage, and measured according to the Bond Area Percentage method set forth herein.

"Cleaning articles" refers to articles for cleaning household surfaces and clothes, such as sweepers or mops, which comprise dry or wet-type disposable cloths typically used for mopping or sweeping lint. Cleaning articles also comprises laundry bags, dryer bags and cleaning sheets.

"Color", as used herein, includes any color in the CIELAB color space including primary color, secondary color, tertiary color, the combination thereof, as well as black and white. As used herein "white" is defined as having $L^* > 90$, $-2 < a^* < 2$, and $-2 < b^* < 2$.

CIE $L^*a^*b^*$ ("CIELAB") is the most commonly used color space specified by the International Commission on Illumination (French Commission internationale de l'éclairage, hence its CIE initialism). It describes all the colors visible to the human eye and was created to serve as a device independent model to be used as a reference.

The three coordinates of CIELAB represent the lightness of the color ($L^* = 0$ yields black and $L^* = 100$ indicates diffuse white; specular white may be higher), its position between red/magenta and green ($a^*$, negative values indicate green while positive values indicate magenta) and its position between yellow and blue ($b^*$, negative values indicate blue and positive values indicate yellow). The asterisk (*) after L, a and b are part of the full name, since they represent $L^*$, $a^*$ and $b^*$, to distinguish them from Hunter's L, a, and b.

"Cross direction", with respect to a web material, refers to the direction along the web material substantially perpendicular to the direction of forward travel of the web material through the manufacturing line in which the web material is manufactured.

"Disposable" is used in its ordinary sense to mean an article that is disposed or discarded after a limited number of usage events over varying lengths of time, for example, less than 20 events, less than 10 events, less than 5 events, or less than 2 events. If the disposable absorbent article is a diaper, a pant, sanitary napkin, sanitary pad or wet wipe for personal hygiene use, the disposable absorbent article is most often disposed after single use.

"Diaper" refers to an absorbent article generally worn by infants and incontinent persons about the lower torso so as to encircle the waist and legs of the wearer and that is specifically adapted to receive and contain urinary and fecal waste. As used herein, term "diaper" also includes "pant" which is defined below.

"Machine direction", with respect to a web material, refers to the direction along the web material substantially parallel to the direction of forward travel of the web material through the manufacturing line in which the web material is manufactured.

"Monocomponent" refers to fiber formed of a single polymer component or single blend of polymer components, as distinguished from Bicomponent or Multicomponent fiber.

"Multicomponent" refers to fiber having a cross-section comprising two or more discrete polymer components, two or more discrete blends of polymer components, or at least one discrete polymer component and at least one discrete blend of polymer components.

"Multicomponent fiber" includes, but is not limited to, "bicomponent fiber." A Multicomponent fiber may have an overall cross section divided into subsections of the differing components of any shape or arrangement, including, for example, coaxial subsections, concentric core-and-sheath subsections, eccentric core-and-sheath subsections, side-by-side subsections, islands-in the sea subsection, segmented pie subsections, etc.

"Multilayered nonwoven web" is a nonwoven web which is made of several fiber layers, wherein the layers have been laid down, on top of one another, in one continuous manufacturing process, wherein the fibers of the multi-layered nonwoven web are consolidated and bonded together to form a self-sustaining web only after the several layers of fibers have been laid down. Hence, the fibers within the different layers have not been substantially bonded together prior to assembling into a multilayered nonwoven web, but instead the fibers of all layers are pattern-bonded together after assembly into a multilayered nonwoven web. However, the individual layers may have undergone a compaction step, typically by passing the layer through the nip between two rollers, or by a roller pressing onto the fibrous layers on top of the moving laydown belt. Such compaction step does not result in the formation of discernible fused bond sites. Laminates made of preformed, self-sustaining webs are consequently not encompassed by the term "multilayered nonwoven web" as used herein.

A "nonwoven web" is a manufactured web of directionally or randomly oriented fibers, consolidated and bonded together by one or more patterns of bonds and bond impressions created through localized compression and/or application of heat or ultrasonic energy, or a combination thereof. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as meltblowing, spunlaid, solvent spinning, electrospinning, and carding. As used herein, "spunlaid" refers to fibers made by spunbond technology without having undergone further processing, such as bonding. The basis weight of nonwoven fabrics is usually expressed in grams per square meter (gsm). For the present invention, a multilayered nonwoven web may be consolidated and bonded by hydroentanglement and/or needle punching, in addition to being consolidated and bonded by bonds obtained by heat and/or compression (including ultrasonic bonding), e.g. in order to impart improved loft to the multilayered nonwoven web.

"Pant", as used herein, refers to disposable garments having a waist opening and leg openings designed for infant or adult wearers. A pant may be placed in position oil the wearer by inserting the wearer's legs into the leg openings and sliding the pant into position about a wearer's lower torso. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).

Disposable absorbent articles

A typical disposable absorbent article comprising the nonwoven web of the present invention is represented in Fig. 1 in the form of a diaper 20.

In more details, Figure 1 is a plan view of an exemplary diaper 20, in a flat-out state, with portions of the structure being cut-away to more clearly show the construction of the diaper 20. This diaper 20 is shown for illustration purpose only as the multilayered nonwoven web of the present invention may be comprised in a wide variety of diapers or other absorbent articles.

As shown in Figure 1, the absorbent article, here a diaper, can comprise a liquid pervious topsheet 24, a liquid impervious backsheet 26, an absorbent core 28 which is preferably positioned between at least a portion of the topsheet 24 and the backsheet 26. The absorbent core 28 can absorb and contain liquid received by the absorbent article and may comprise superabsorbent polymer 60. The diaper 20 may also include optionally an acquisition system with an upper and lower acquisition layer (52 and 54).

The diaper may also comprise elasticized leg cuffs 32 and barrier leg cuffs 34, and a fastening system, such as an adhesive fastening system or a hook and loop fastening member, which can comprise tape tabs 42, such as adhesive tape tabs or tape tabs comprising hook elements, cooperating with a landing zone 44 (e.g. a nonwoven web providing loops in a hook and loop fastening system). Further, the diaper may comprise other elements, which are not represented, such as a back elastic waist feature and a front elastic waist feature, side panels or a lotion application.

The diaper 20 as shown in Figure 1 can be notionally divided in a first waist region 36, a second waist region 38 opposed to the first waist region 36 and a crotch region 37 located between the first waist region 36 and the second waist region 38. The longitudinal centerline 80 is the imaginary line separating the diaper along its length in two equal halves. The transversal centerline 90 is the imagery line perpendicular to the longitudinal line 80 in the plane of the flattened out diaper and going through the middle of the length of the diaper. The periphery of the diaper 20 is defined by the outer edges of the diaper 20. The longitudinal edges of the diaper may run generally parallel to the longitudinal centerline 80 of the diaper 20 and the end edges run between the longitudinal edges generally parallel to the transversal centerline 90 of the diaper 20.

The chassis 22 of the diaper 20 comprises the main body of the diaper 20. The chassis 22 comprises the absorbent core 28 and preferably an outer covering including the topsheet 24 and/or the backsheet 26. The majority of diapers are unitary, which means that the diapers are formed of separate parts united together to form a coordinated entity so that they do not require separate manipulative parts like a separate holder and/or liner.

The chassis 22 comprises the main structure of the diaper with optional other features such as back ears 40, front ears 46 and/or barrier cuffs 34 attached to form the composite diaper structure. The topsheet 24, the backsheet 26, and the absorbent core 28 may be assembled in a variety of well known configurations, in particular by gluing or heat embossing. Exemplary diaper configurations are described generally in US3,860,003; US5,221,274; US5,554,145; US5,569,234; US5,580,411; and US6,004,306.

The diaper 20 may comprise leg cuffs 32 which provide improved containment of liquids and other body exudates. Leg cuffs 32 may also be referred to as leg bands, side flaps, barrier cuffs, or elastic cuffs. Usually each leg cuffs will comprise one or more elastic string 33, represented in exaggerated form on Fig. 1 comprised in the chassis of the diaper for example between the topsheet and backsheet in the area of the leg openings to provide an effective seal while the diaper is in use. It is also usual for the leg cuffs to comprise "stand-up" elasticized flaps (barrier leg cuffs 34) which improve the containment of the leg regions.

Of course, it will be recognized that any disposable absorbent article design may comprise one or more multilayered nonwoven webs of the present invention. The disclosure above is merely for illustrative purposes.

Multilayered nonwoven webs

The present invention is directed towards a multilayered nonwoven web comprising at least a first and a second layer, wherein the first layer forms the first outer surface of the multilayered nonwoven web. The fibers of the first layer and the fibers of the second layer differ from each other in color. As used herein, "difference in color" or "to differ in color" includes different colors (such as blue and green) as well as different shades of the same color (such as lighter blue and darker blue). Also, as used herein, white, black, and shades of grey are considered as colors. The difference in color between the fibers of the first and second layer needs to be large enough to obtain a delta E* of at least 0.7 or at least 1.0 between bonded and unbonded areas of the multilayered nonwoven web after the multilayered nonwoven web has been pattern bonded (as explained in more detail below). The difference in color may be obtained by using different pigmentation for the fibers of the first and second layer.

The multilayered nonwoven web is pattern bonded. Typically, the bond pattern is imparted by use of heat and/or pressure, and/or by ultrasonic bonding. Due to the use of heat, pressure and/or ultrasonic energy , the fibers of the different layers in the bond areas are pressed tightly together, which results in plastic deformation of the fibers. Especially if bonding is achieved by heat, or by heat and compression, the fibers in the bonded areas are molten, completely or partially, such that in the bonded areas the individual fibers of the different layers are fused together (coalescence) to form a film-like structure. In the bonded areas, the deformed/fused fibers comprise the material of the fibers of the first and second layer (and of the optionally further layers).

Due to this bonding, the fibers of the layers below the layers forming the outer surface become more discernible from the outside of the multilayered nonwoven web in the bonded areas. When viewing the first outer surface of the multilayered nonwoven web, the visual appearance of the unbonded areas is mainly determined by the visual appearance of the fibers of the first, outer layer, while the visual appearance of the bonded areas is determined by both, the fibers of the first, outer layer as well as by the fibers of the second layer and by the fibers of the optional further layers. In the unbonded areas, the fibers of the first layer and optional further layers positioned above the second layer (when viewed from the first outer surface) refract the light which "masks" the fibers of the second layer. This masking effect is generally increased with increased level of pigment(s) and/or for fibers having small diameter which is closer to the wavelength of the visible light spectrum (i.e. especially fiber diameters of close to $1.0\mu m$ or smaller). In the bonded areas, the fibers are deformed and pressed or fused together such that the bonded areas do not have individual fibers which might diffract light refraction and the fused fiber mass creates a distinct visual appearance. Without wishing to be bound by theory, it is also believed that a certain level of interlayer mixing may occur in the bonded areas due to compression / fusion of fibers, which improves the distinct color appearance in the bonded areas as the second layer is pigmented differently from the first layer.

The fibers of the first and second layer and the fibers of the optional further layers are selected such that the difference in color results in the bonded areas of the first outer surface having a first color and the unbonded areas of the first outer surface having a second color which is different from the first color. On the first outer surface, the delta E* between the bonded areas and the unbonded areas is at least 0.7 as determined by the Test Method disclosed herein below. The delta E* between the bonded areas and the unbonded areas may be at least 1.0 or at least 2.0, or at least 2.5, or at least 3.0, or at least 4.0, or at least 5.0, or at least 10 or at least 15. It has been found that a delta E* of 0.7 is already sufficient to obtain bond areas which are visually distinct from the unbonded areas, such that the visual difference is noticeable to the naked eye. However, as delta E* increases, the bonded areas become more distinguishable and more pronounced versus the unbonded areas.

Further color distinctions can be found using delta C* and delta H*. It has been found that a sufficient and significant color distinction can be achieved by providing a multilayered nonwoven web which may have delta C* of at least 1, or at least 3 or at least 5 between the bonded areas and the unbonded areas. Also, sufficient and significant color distinction can be achieved by providing a multilayered nonwoven web which may have delta H* of at least 1, or at least 1.2, or at least 1.5, or at least 2 between the bonded areas and the unbonded areas.

Notably, in the multilayered nonwoven webs of the present invention, the areas having different color are congruent with the bonded areas, as the different color is obtained simultaneously with introducing the bond pattern. Hence, by simply imparting a print on a surface of a multilayered nonwoven web to "mimic" the effect of the present invention, the same visual effect can only be achieved if the print is exactly registered with the bonded areas, thus requiring a very exact and demanding manufacturing process. However, even if using such cost-efficient and complex manufacturing process, the resulting multilayered nonwoven web would still have the drawbacks mentioned above, e.g. increases risk of rub-off or wash-off of the print during use. For the present invention, the delta E* between the bonded and the unbonded areas of at least 0.7, or at least 1.0, or at least 2.5, or at least 3.0, or at least 4.0, or at least 5.0, or at least 10 or at least 15 is obtained by the difference in color between the different layers of the multilayered nonwoven web and is not obtained by providing a print (which corresponds with the bond pattern) on a surface of the multilayered nonwoven web.

Bonded areas are typically areas of reduced thickness in a nonwoven web (as the fibers have been compressed and/or fused together). It has been found that the congruence between difference in color and difference in thickness of the multilayered nonwoven web results in a multilayered nonwoven web which is perceived as having enhanced 3-dimensional appearance and, e.g. if used as topsheet for absorbent articles, as having good fluid handling properties (suggesting fast liquid uptake).

The multilayered nonwoven web comprises at least a first and a second layer. The multilayered nonwoven web may consist only of the first and second layer. However, the multilayered nonwoven web may comprise further layers, such that the multilayered nonwoven web comprises more than two layers (e.g. the multilayered nonwoven web may consist of three, four, five or more than five layers).

The first layer of the multilayered nonwoven web forms the first outer surface of the multilayered nonwoven web. In embodiments wherein the multilayered nonwoven web comprises more than a first and a second layer, the second layer may be in direct contact with first layer. Alternatively, the second layer may not be in direct contact with the first layer but instead, one or more additional layers are provided between the first and the second layer. The second layer may or may not form the second outer surface of the multilayered nonwoven web (however, if the multilayered nonwoven web consists only of the first and second layer, the second layer will naturally form the second outer surface).

Suitable multilayered nonwoven webs useful in the present invention comprise spunlaid layers, meltblown layers and layers of nanofibers. Generally, the diameter of spunlaid fibers is larger compared to the diameter of meltblown fibers, which in turn have a somewhat larger diameter than nanofibers. Spunlaid fibers typically have a diameter of $10\mu m$ to $30\ \mu m$; meltblown fibers have a diameter of $0.5\ \mu m$ to $\leq 10\ \mu m$, while nanofibers generally have a diameter of $0.01\ \mu m$ to $1.5\ \mu m$. Nanofibers can be made by different processes, including advanced meltblown as disclosed in U.S. 7,922,943B2, melt film fibrillation as disclosed in U.S. 7,931,457B2 or electrospinning as disclosed in U.S. 6,616,435B2. The multilayered nonwoven web may also be made of layers of carded fibers (so-called staple-fibers) or the multilayered nonwoven web may comprise one or more layers of carded fibers and one or more layers of spunlaid, meltblown and/or nano fibers. Examples include, but are not limited to SMS multilayered nonwoven webs, comprising a spunlaid, a melt-blown and a further spunlaid layer. Another suitable multilayered nonwoven web of the present invention comprises a SMMS- structure (two outer spunlaid layers and two inner meltblown layers) or a SMMMS-structure (two outer spunlaid layers with three inner meltblown layers). Other suitable multilayered nonwoven webs are SNS materials, comprising a spunlaid, a nanofiber and a further spunlaid layer, or SMNS materials, comprising a spunlaid, a meltblown, a nanofiber and a further spunlaid layer.

Multilayered nonwoven webs having spunlaid layers forming the outer surfaces of the multilayered nonwoven web tend to have better resistance to fuzz, i.e. the fibers exposed to the surface of the multilayered nonwoven web are not as easily abraded and twitched out of the multilayered nonwoven web as fine fibers with smaller diameters (such as meltblown fibers or nanofibers). This may be especially beneficial when the multilayered nonwoven web forms at least a part of the garment-facing surface of a disposable absorbent article, such as a diaper, where the garment-facing surface is rubbed against clothes or other items (such as carpets) when the article is worn.

On the other hand, multilayered nonwoven webs, wherein the outer surface is formed of a meltblown layer or layer of nanofibers may be able to provide a more uniform appearance on the outer surface at a given basis weight of the fiber layer as the fibers have a considerably smaller diameter. Hence, the fibers of the first layer can more readily "mask" the fibers of the second layer (and optional additional layers) in the unbonded areas.

To enable both, good surface abrasion resistance and good masking of the fibers comprised by the second layer, the multilayered nonwoven web may have a first layer made of spunlaid fibers and an additional (third) layer made of meltblown fibers or nanofibers, the additional (third) layer being positioned between the first and second layer. For such multilayered nonwoven webs, the first layer and the additional (third) layer may have white color.

At least one of the first or second layers of the multilayered nonwoven web may be formed of thermoplastic fibers, such as polyolefin. However, especially if the difference in color between the fibers of the first and second layer is relatively small, both, the first and second layer of the multilayered nonwoven web may be formed of thermoplastic fibers. If the multilayered nonwoven web comprises one or more additional layers, one or more of these additional layers may also be formed of thermoplastic fibers. In one embodiment, all layers of the multilayered nonwoven web are formed of thermoplastic fibers.

However, as long as a sufficient number of thermoplastic fibers are comprised in the bonded areas, the bond areas (and hence the multilayered nonwoven web as a whole) may comprise layers which are not formed of thermoplastic fibers. Hence, one or more layers of the multilayered nonwoven web may be made of non-thermoplastic fibers, such as natural fibers.

Generally, the multilayered nonwoven web may be formed from one or more thermoplastic polymers. Suitable non-woven fiber materials may include, but are not limited to polymeric materials such as polyolefins, polyesters, polyamide or specifically polypropylene (PP), polyethylene (PE), poly-lactic acid (PLA), polyethylene terephthalate (PET), Nylon 6-6 as well as combinations thereof (such as blends and copolymers thereof). Resins including PP may be particularly useful because of polypropylene's relatively low cost and surface friction properties of fibers formed from it (i.e., they have a relatively smooth, slippery tactile feel). Resins including PE may also be desirable because of polyethylene's relative softness/pliability and even more smooth/slippery surface friction properties. Relative each other, PP currently has a lower cost and fibers formed from it have a greater tensile strength, while PE currently has a greater cost and fibers formed from it have a lower tensile strength but greater pliability and a more smooth/slippery feel.

Accordingly, it may be desirable to form multilayered nonwoven web fibers from a blend of PP and PE resins, finding a balance of the best proportions of the polymers to balance their advantages and disadvantages. In some examples, the fibers may be formed of PP/PE blends such as described in U.S. Pat. No. 5,266,392.

The fibers of all or some of the layers of the multilayered nonwoven web may be solid fibers having a round cross-section. Alternatively, the fibers of all or some of the layers may be hollow fibers and/or may have a shaped, i.e. non-round cross-section, such as a multilobal cross-section. The multilayered nonwoven web may comprise one or more layer of solid fibers and one or more other layers of hollow fibers.

The fibers of all or some of the layers of the multilayered nonwoven web may be monocomponent fibers. Alternatively, the fibers of all or some of the layers may be multicomponent fibers, such as bicomponent fibers. The multilayered nonwoven web may comprise one or more layer of monocomponent fibers and one or more other layers of multicomponent fibers such as bicomponent fibers.

The multilayered nonwoven web may be formed of any basis weight. However, relatively higher basis weight, while having relatively greater apparent caliper and loft, also has relatively greater cost. On the other hand, the basis weight of the multilayered nonwoven web has to be high enough such that the individual layers have a sufficiently high amount of fibers to enable a multilayered nonwoven web wherein the delta E* between bonded areas and unbonded areas is at least 0.7. Suitable basis weight for nonwovens of the present invention have been found to be 200 gsm or less, or from 7 gsm to 70 gsm, or from 10 gsm to 50 gsm, or from 12 gsm to 30 gsm. The basis weight of the first layer may be from 1 gsm to 10 gsm, or from 2 gsm to 10 gsm, or form 3 gsm to 8 gsm. The basis weight of the second layer may also be from 1 gsm to 100 gsm, or from 2 gsm to 50 gsm, or form 3 gsm to 10 gsm.

The suitable basis weight of the individual layers also depends on the types of fibers which are used and on the presence or absence of additional fiber layers. If the first layer is made of spunlaid fibers, the basis weight may be from 5 gsm to 50 gsm if no additional (third) layer is placed between the first layer and the second layer (i.e. if the fibers of the second layer are solely concealed by the fibers of the first layer in the unbonded areas). If the fibers of the first layer are made of meltblown or nanofibers, and/or if additional layer(s) are placed between the first and second layer, the basis weight of the first layer may be from 1 gsm to 50 gsm.

Generally, to obtain the desired degree of difference in color between the bonded areas and the unbonded areas in the multilayered nonwoven web of the present invention, the layers overlying the second layer -when viewed from the first outer surface- should have a sufficient basis weight such that the shine through of the color of the second layer through the unbonded areas of the layers laying above the second layer is reduced. Thus, the combined basis weight of all layers lying on top of the second layer when viewed from the first outer surface may be at least 5 gsm, or should be from 1 gsm to 100 gsm, or from 3 gsm to 50 gsm or from 5 gsm to 40 gsm or from 8 gsm to 30 gsm. In embodiments, wherein only the first layer is lying on top of the second layer when viewed from the first outer surface, the basis weight of the first layer may be at least 5 gsm, or should be from 5 gsm to 100 gsm, or from 8 gsm to 50 gsm. If the multilayered nonwoven web has further layer lying on top of the second layer in addition to the first layer when viewed from the first outer surface, the basis weight of the first layer may be less than 3 gms, as long as the combined basis weight of all layers lying on top of the second layer is at least 5 gsm. However, in such embodiments it may be desirable that the additional layers lying on top of the second layer have the same or at least a similar color than the first layer, such as white color

Lower basis weights of the layers overlying the second layer -when viewed from the first outer surface - can provide the desired masking effect (and hence, ultimately the required delta E* between bonded and unbonded areas) if the fibers are sufficiently colored, e.g. by using sufficient amounts of white pigments, such as titanium dioxide.

Thus, by selecting the appropriate combination between basis weight, the degree of pigmentation and the types of fibers (such as spunlaid or meltblown) for the fiber layers overlying the second layer, sufficient masking can be obtained. Generally, for good masking properties, the first layer and the optional one or more additional layers positioned between the first layer and the second layer together may have an opacity greater than 40%, or greater than 45% or greater than 60% as measured according to the test method set out herein below.

The first layer forming the first outer surface of the multilayered nonwoven web may comprise a white pigment. Thereby, the first layer can provide good opacity to conceal the second layer and the optional further layers underneath the first layer in the unbonded areas. One example of a suitable white pigment is titanium dioxide ($TiO_2$). $TiO_2$ provides brightness and relatively high refractive index. It is believed that addition of $TiO_2$ to the polymer(s) from which the fibers may be formed, in an amount up to 5.0% by weight of the first layer of the nonwoven, may be effective to achieve the desired results. However, because $TiO_2$ is a relatively hard, abrasive material, inclusion of $TiO_2$ in amounts greater than 5.0% by weight may have deleterious effects, including wear and/or clogging of spinnerets; interruption and weakening of the structure of the fibers and/or calendar bonds there between; undesirably increasing the surface friction properties of the fibers (resulting in a less smooth tactile feel); and unacceptably rapid wear of downstream processing equipment components. While 5.0% by weight $TiO_2$ may be an upper limit, it may be more desirable to include no more than 4.0% or no more than 3.0% or no more than 2.0% by weight $TiO_2$ in the first layer.

The fibers of the first layer and the fibers of the second layer differ from each other in pigmentation.

As used herein, to "differ in pigmentation" or "difference in pigmentation" means

a) the fibers of the first layer comprise a pigment which is different from the pigment of the second layer; or

b) the fibers of the first layer comprise a different combination of pigments; or

c) the fibers of the first layer comprise different amounts of the same pigment(s) versus the second layer; or

d) combinations of any of options a) to c).

A pigment is a material, which can be organic or inorganic. A pigment changes the color of reflected or transmitted light as the result of wavelength-selective absorption. This physical process differs from fluorescence, phosphorescence, and other forms of luminescence, in which a material emits light. A pigment is a generally insoluble powder, which differs from a dye, which either is itself a liquid or is soluble in a solvent (resulting in a solution). Dyes are often used to provide a print on the surface of a nonwoven web, such as graphics, pattern or images. Hence, these dyes do not form a part of the fibers of the nonwoven web but are rather applied on the web surface. Contrary thereto, in the present invention the pigments are comprised within the fibers of the multilayered nonwoven web, which eliminates the risk of rub-off or wash-off of the colour(s) imparted to the multilayered nonwoven web by the pigment.

For the present invention, the pigment will typically be mixed with the thermoplastic material, of which the fibers are made. Often, the thermoplastic material is colored in a so-called masterbatch, wherein a large quantity of thermoplastic material is molten and colored with one or more pigments. The homogeneously colored thermoplastic material is then solidified and typically formed into pellets, which can be used for the manufacture of the multilayered nonwoven webs (or individual layers of a multilayered nonwoven web). Colored masterbatches useful for the present invention are Lufilen and Luprofil supplied by BASF; Remafin for polyolefin fibers, Renol-AT for polyester fibers, Renol-AN for polyamide fibers and CESA for renewable polymers supplied by Clariant. Hence, the pigment will be suspended in the molten thermoplastic material prior to the thermoplastic material being forced through the spinnerets to form and lay down the fibers which form the nonwoven web.

Fillers are particles which are often added to materials, such as the fibers of nonwoven webs, to lower the consumption of more expensive materials, such as the thermoplastic material of the fibers (e.g. polyethylene, polypropylene). However, the fillers may also be more expensive than the thermoplastic materials of the fibers and may be used to impart desired properties to the fibers and the resulting nonwoven webs or to improve the process ability of thermoplastic material (such as reduction of melt viscosity). For the present invention, the fillers may be selected to impart or at least contribute to the difference in color between the bonded areas and the unbonded areas of the multilayered nonwoven web. Filler particles can be organic or inorganic. A typical example of filler is titanium dioxide, which can impart a white color to the multilayered nonwoven web. Another example is calcium carbonate ($CaCO_3$), which can also provide for a higher opacity. For the present invention, fillers are considered as pigments, as long as it is able to impart a color to multilayered nonwoven web (such as the white color imparted by titanium dioxide).

The first layer of the multilayered nonwoven web may desirably serve as kind of a masking layer to conceal the non-white color of the second layer and optional further layers underneath. It has been found that hollow fibers, nanofibers and fibers comprising at least 0.5 percent by weight of a white pigment, are especially useful to provide good masking properties. Hence, the fibers of the first layer may comprise or consist of hollow fibers, nanofibers, fibers comprising at least 0.5 percent by weight of a white pigment.

Generally, it may be desirable that the fibers of the first layer have a lighter color than the fibers of the second layer (i.e. the color has a higher L* value), such that the bonded areas take a more intense and/or darker color compared to the unbonded areas.

The second layer of the multilayered nonwoven web may be of non-white color. The fibers of the second layer may thus comprise a non-white pigment, such as a blue pigment, a yellow pigment or a green pigment. The fibers of the second layer may be meltblown fibers, spunlaid fibers, or staple fibers.

The multilayered nonwoven web may comprise an additional third layer. The third layer may be positioned between the first and second layer. In such embodiments, the third layer may be in direct contact with the first and second layer or, if the multilayered nonwoven web comprises further layers in addition to the first, second and third layer, the third layer may be in direct contact with only the first layer or only the second layer. Alternatively, the third layer may form the second outer surface of the multilayered nonwoven web.

If the third layer is positioned between the first and second layer, the fibers of the third layer may be meltblown or nanofibers to increase masking of the fibers of the second layer when viewed from the first surface (hence the third layer is an additional "masking layer" further to the first layer). If the third layer is positioned below the second layer and forms the second outer surface, the fibers of the third layer may be spunlaid fibers to provide improved abrasion resistance to the second outer surface (hence, the third layer is an outer surface-forming layer). A third layer forming the second outer surface of the multilayered nonwoven web may be especially advantageous if the multilayered nonwoven web is visible from both surfaces in use, e.g. when it is used in a disposable absorbent article as back ear material.

The multilayered nonwoven web may comprise a third layer (such as a meltblown or nanofiber layer) which is a masking layer and is placed between the first and second layer, and may further comprise a fourth layer (such as a spunlaid fiber layer), which is an outer surface-forming layer which forms the second outer surface. Generally, the first, third, and fourth layer may have the same or substantially the same color, such as white.

Also, the multilayered nonwoven web may comprise a fifth layer (such as a meltblown or nanofiber layer) which may, for example, be positioned between the second layer and the fourth layer to provide additional masking of the second layer fibers to the multilayered nonwoven web, when viewed from the second outer surface. In these embodiments it may be desirable that the fifth layer has the same or substantially the same color as the fourth (second outer surface-forming) layer. Generally, the first, third, fourth and fifth layer may have the same or substantially the same color, such as white. Such embodiments comprising five layers are especially beneficial when the multilayered web is used as a component of an absorbent article, which is visible to the consumer from both outer surfaces (e.g. when the multilayered nonwoven web is used as back and/or front ear material and/or as elasticized leg cuff and/or barrier leg cuff).

If the multilayered nonwoven web comprises one or more further layer(s) in addition the first and second layers, the one or more additional layer(s) may differ in color from the first and second layer or they may have the same color as either the first layer or the second layer. Thus, the additional layer(s) may differ in pigmentation from the first and second layer. Alternatively, the additional layer(s) may have the same pigmentation as the first or second layer. In a still further alternative, the additional layer(s) may be free from pigmentation.

If the multilayered nonwoven web comprises more than one further layer in addition to the first and second layer, these additional layers may all have similar color (i.e. similar pigmentation). Alternatively, the additional layers differ from each other with regard to their pigmentation. Also, one or more additional layer(s) may pigmentation while one or more other additional layer(s) are free from pigmentation.

The fibers of the additional layer(s) may be meltblown fibers, spunlaid fibers, nanofibers or staple fibers. Different additional layers may be made of different fibers, e.g. a third layer may be made of meltblown fibers while a fourth layer is made of nanofibers. Alternatively, the additional layers may all be made of the same type of fiber, e.g. all are made of spunlaid fibers, or all are made of meltblown fibers.

Given the many possible variations when combining different first and second layers and optional further layers, it is apparent that the present invention allows for numerous possible embodiments with all kinds of color combinations of the bonded areas and the unbonded areas so that a large variety of different multilayered nonwoven webs can be obtained.

The multilayered nonwoven web of the present invention is pattern bonded. As used herein, the term "pattern bonded" comprises a plurality of individual bonded areas (which may be arranged as a repeating pattern) which are surrounded by continuous unbonded areas, as well as a continuous bonded area which surrounds a plurality of individual unbonded areas. Also, the term "pattern bonded" comprises bonded areas and unbonded areas which are alternating with each other, e.g. as stripes or waves extending in machine direction or cross-direction. The overall bonded area, i.e. the sum of all bonded areas taken together, should be from 5% to 80% of the overall area of the multilayered nonwoven web, or from 5% to 50%, or from 10% to 40% of the overall area of the multilayered nonwoven web. The overall bonded area may be determined by the Test Method on Bond Area Percentage below. However, if the bonded areas are introduced by calendar bonding, the percentage of the raised areas on the pattern roll with regard to the overall surface area of the patterned calendar roll can be taken as the bonded area.

If the multilayered nonwoven web has a plurality of individual bonded areas, the size of the individual bonded areas comprised by the bond pattern of the multilayered nonwoven web may be at least 0.3 mm$^2$, or at least 0.4 mm$^2$ or at least 0.5 mm$^2$ or at least 0.7 mm$^2$; they may also be no more than 10 mm$^2$ or no more than 5 mm$^2$. If individual bonded areas are not all similar in size, the size of all individual bonded areas may be in the range of from 0.3 mm$^2$ to 5 mm$^2$ or from 0.4 mm$^2$ to 5 mm$^2$ or from 0.5 mm$^2$ to 5 mm$^2$. Generally, for multilayered nonwoven webs wherein delta E* between the bonded and unbonded areas is relatively large (such as greater than 5, or greater than 10), smaller individual bonded areas (such as 0.3 mm$^2$ to 0.6 mm$^2$) may be easily visible to the naked eye, whereas for a smaller delta E* (such as smaller than 5) it may be desirable to have slightly larger individual bonded areas (such as larger than 0.6 mm$^2$). Also, for multilayered nonwoven webs having a relatively high basis weight (such as greater than 12 gsm, or greater than 15 gsm), smaller individual bonded areas (such as 0.3 mm$^2$ to 0.7 mm$^2$) may be easily visible to the naked eye, whereas for a lower basis weights (such as smaller than 15 gsm, or smaller than 12 gsm) it may be desirable to have slightly larger individual bonded areas (such as larger than 0.6 mm$^2$). Bond pattern designs that can be easily perceived by the consumer to communicate design attributes are preferred. A "consumer noticeable pattern" as used herein is a pattern with an area of at least 0.6 mm$^2$ or at least 0.9 mm$^2$, preferably more than 2 mm$^2$ with bonding area percentage of at least 5 % or at least 10 %. The bonding area percentage may be less than 50%, preferably less than 25%; more preferably less than 15%.

The multilayered nonwoven web of the present invention can be used in an absorbent article, such as the disposable diaper described above. For example, the multilayered nonwoven web may form a portion of or the whole of the topsheet, the backsheet, the back ears, the front ears, the fastening system (such as at least a portion of the landing zone or the fastening tapes), the elasticized leg cuffs and/or the barrier leg cuffs. If the multilayered nonwoven web forms a portion or the whole of the topsheet of an absorbent article, the first surface of the multilayered nonwoven web may face towards the skin of the wearer when the article is in use. If the multilayered nonwoven web forms a portion or the whole of the backsheet, the back ears, the front ears, the landing zone and/or the fastening tapes, the first surface of the multilayered nonwoven web may face towards the garment of the wearer when the article is in use.

The multilayered nonwoven web can be made by the following method:
A first fibrous layer is laid down on a support member, such as a belt or a drum. The fibers may be made of molten thermoplastic material which is processed into fibers by a suitable method as is well known in the art.

A second fibrous layer is laid down on top of the first fiber layer. These fibers may also be made of molten thermoplastic material.

Alternatively, the second fibrous layer may be laid down prior to laying down the first fibrous layer, such that the first layer is laid down onto the second layer.

The fibers of the first layer and fibers of the second layer differ from each other in color, e.g. by differing from each other in pigmentation.

After being laid down on top of one another, the first and second layers are bonded to each other by a bond pattern. Bonding may be achieved by the application of heat, pressure, ultrasonic energy or combinations thereof.

Once the bonding pattern is applied, the bonded areas of the first surface have a first color and the unbonded areas of the first surface have a second color which is different from the first color. On the first outer surface, the delta E* between the bonded areas and the unbonded areas is at least 0.7, or at least 1.0, or at least 2.5, or at least 3.0, or at least 4.0, or at least 5.0, or at least 10 or at least 15.

One, more than one or all layers of the multilayered nonwoven webs of the present invention may be formed from any suitable resins by conventional processes, in which the resin(s) are heated and forced under pressure through spinnerets. The spinnerets eject fibers of the polymer(s), which are then directed onto a moving belt; as they strike the moving belt they are laid down in somewhat random orientations to form a spunlaid batt. The batt then -after all the layers of the multilayered nonwoven web have been laid down- may be calendar-bonded to form the nonwoven web. However, other well known manufacturing techniques, such as carding staple fibers, may also be used to make one, more than one or all layers of the multilayered nonwoven webs of the present invention.

The bond pattern may be imparted to the multilayered nonwoven web by heat, pressure or a combination of heat and pressure as well as by using ultrasonic bonding and by combinations of heat, pressure and/or ultrasonic bonding. A suitable technique to provide the bond pattern is by calendar-bonding. Calendar bonding may be accomplished by passing the multilayered nonwoven material through the nip between a pair of rotating calendar rollers, thereby compressing and consolidating the fibers to form the multilayered nonwoven web. One or both of the calendar rollers may be heated, so as to promote plastic deformation, intermeshing and/or thermal bonding/fusion between superimposed and closely neighboring fibers compressed at the nip. The calendar rollers may form operable components of a bonding mechanism in which they are urged together by a controllable amount of force, so as to exert the desired compressing force/pressure at the nip. In some processes heating may be deemed unnecessary, since compression alone may generate sufficient energy within the fibers to effect bonding, resulting from rapid deformation and frictional heat generated in the fibers as they are urged against each other where they are superimposed and closely neighboring, resulting in plastic deformation and intermeshing, and possibly thermal bonding/fusion. In some processes an ultrasonic energy source may be included in the bonding mechanism so as to transmit ultrasonic vibration to the fibers, again, to generate heat energy within them and enhance bonding. One or both of the calendar rollers may have their circumferential surfaces machined, etched, engraved or otherwise formed to have thereon a pattern of protrusions and recessed areas, so that bonding pressure exerted on the nonwoven material at the nip is concentrated at the outward surfaces of the protrusions, and reduced or substantially eliminated at the recessed areas. As a result, an impressed pattern of bonds between fibers forming the multilayered nonwoven web, generally corresponding to the pattern of protrusions on the calendar roller, is formed on the multilayered nonwoven web. One calendar roller may have a smooth, unpatterned cylindrical surface, and the other may be formed with a pattern as described; this combination will impart a pattern on the web generally reflecting the pattern on the formed calendar roller. In some examples both calendar rollers may be formed with patterns, and in particular examples, differing patterns that work in combination to impress a combination pattern on the web such as described in, for example, U.S. Pat. No. 5,370,764.

In embodiments, wherein one calendar roll is formed with protrusions and the other calendar roll has a substantially smooth outer surface, it is preferred that the multilayered nonwoven web is pattern bonded such that the first outer surface gets in direct contact with the calendar roll formed with protrusions.

A repeating pattern of protrusions and recessed areas, may be formed onto one calendar roller. For example, the protrusions on the calendar roll may be rhombus-, diamond-, or otherwise shaped raised surfaces of protrusions, while the areas between them represent recessed areas. Without intending to be bound by theory, it is believed that the visual impact of the bond impressions impressed on the multilayered nonwoven web, as well as the tensile strength, resulting from the protrusion surfaces, may be affected by the area of the protrusion surfaces. Accordingly, it is believed desirable that the area of the individual protrusion surfaces be from at least 0.3 mm$^2$, or at least 0.4 mm$^2$ or at least 0.5 mm$^2$ or at least 0.7 mm$^2$; they may also be no more than 10 mm$^2$ or no more than 5 mm$^2$. If the individual protrusion areas are not all similar in size, the size of all individual protrusion areas may be in the range of from 0.3 mm$^2$ to 5 mm$^2$ or from 0.4 mm$^2$ to 5 mm$^2$ or from 0.5 mm$^2$ to 5 mm$^2$, Protrusion surfaces may have diamond shapes as shown, or may have any other suitable shape, although it is believed that a diamond, rectangle, square or oval shape may have the desirable effect of simulating the appearance of stitching, as in a quilt.

Protrusion surfaces on the calendar roll may be arranged such that they substantially circumscribe a repeating pattern of recessed areas. Thus, the recessed areas are island-like areas which are surrounded by a continuous area of protrusions (i.e. raised areas). The, resulting, multilayered nonwoven web will then have a continuous bonded areas which delimits a plurality of individual unbonded areas. The recessed areas on the calendar roll may be in the form of geometric shapes. The geometric shapes may be diamonds or squares, or may have other shapes, including but not limited to triangles, diamonds, parallelograms, other polygons, circles, hearts, moons, etc.

Test methods

Measurement of delta E*

Bonded versus unbonded area color difference analysis

The bonded pattern color difference measurement is based on the CIE L* a* b* color system (CIELAB). A flat bed scanner capable of scanning a minimum of 24 bit color at 1200 dpi and has manual control of color management (a suitable scanner is an Epson Perfection V750 Pro from Epson America Inc., Long Beach CA) is used to acquire images. The scanner is calibrated against a color reflection target compliant to ANSI method IT8.7/2-1993 using color management software (a suitable package is MonacoEZColor available from X-Rite Grand Rapids, MI) to construct a scanner profile. The resulting calibrated scanner profile is opened within an imaging program that supports sampling in CIE L* a* b* (a suitable program is Photoshop S4 available from Adobe Systems Inc., San Jose, CA) to measure bonded and unbonded areas.

Turn on the scanner for 30 minutes prior to calibration. Place the IT8 target face down onto the scanner glass and close the scanner lid. Open the MonacoEZColor software and select acquire image using the Twain software included with the scanner. Within the Twain software deselect the unsharp mask setting and any automatic color correction or color management options that may be included in the software. If the automatic color management cannot be disabled, the scanner is not appropriate for this application. Acquire a preview scan at 200 dpi and 24 bit color. Insure that the scanned image is straight and first outer surface facing side-up. Crop the image to the edge of the target, excluding all white space around the target, and acquire the final image. The MonacoEZColor software uses this image to compare with included reference files to create and export a calibrated color profile compatible with Photoshop. After the profile is created the scan resolution (dpi) can be changed, but all other settings must be kept constant while imaging samples.

Identify the first outer surface of the multilayered nonwoven web that contains the bonded areas of interest. Remove a piece of the multilayered nonwoven web. For convenience of handing, the sample size may be a 75 mm by 75 mm piece, however, as will be appreciated by the person skilled in the art, smaller samples sizes can be used. If the multilayered nonwoven web needs to be removed from a product, such as an absorbent article, it may be necessary to use a cryogenic freeze spray (e.g. CytoFreeze, Control Company, TX) to remove the specimen from the product. Precondition samples at about 23°C $\pm$ 2 C° and about 50% $\pm$ 2% relative humidity for 2 hours prior to testing.

Open the scanner lid and place the specimen onto the scanner glass with the first outer surface facing the glass. Cover the specimen with the white background (in this test method white is defined as having L* > 94, -2 < a* < 2, and -2 < b* < 2) and close the lid. Acquire and import a scan of the specimen into Photoshop at 600 dpi and 24 bit color. Assign the calibrated scanner profile to the image and change the mode to Lab Color ("Lab Color" in Photoshop corresponds to the CIE L* a* b* standard). Select the "eyedropper" color selection tool. Set the sampling size of the tool to include as many pixels as possible within a bonded area without including pixels from adjacent unbonded areas. Using the eyedropper tool measure and record L* a* b* values in 10 different bonded areas in the nonwoven image. Average the 10 individual L* a* b* values and record as $L_1$, $a_1$, and $b_1$ respectively. Repeat the measure in like fashion for 10 different unbonded areas in the nonwoven image, and record the averaged values as $L_2$, $a_2$ and $b_2$. Calculate and report the color difference (delta E*) between the bonded and unbonded areas using the following equation:

$$delta\ E^* = \sqrt{(L_2^* - L_1^*)^2 + (a_2^* - a_1^*)^2 + (b_2^* - b_1^*)^2}$$

and report to the nearest 0.01 units. A total of three substantially identical nonwoven webs are measured for each sample set. Average the three delta E** values and report to the nearest 0.1 unit.

Other color analyses that may be useful are made using the calculations of delta Chroma (delta C*) and delta Hue (delta H*).

$$Delta\ C^* = square\text{-}root({a^*_1}^2 + {b^*_1}^2) - square\text{-}root({a^*_2}^2 + {b^*_2}^2)^2$$

$$Delta\ H^* = square\text{-}root[(a^*_2 - a^*_1)^2 + (b^*_2 - b^*_1)^2 - (DeltaC^*)^2]$$

Image Analysis of Bond Impressions

Area and distance measurements are performed on images generated using a flat bed scanner capable of scanning at a resolution of at least 4800 dpi in reflectance mode (a suitable scanner is the Epson Perfection V750 Pro, Epson, USA). Analyses are performed using ImageJ software (Vs. 1.43u, National Institutes of Health, USA) and calibrated against a ruler certified by NIST.

Identify the first outer surface of the multilayered nonwoven web that contains the bonded areas of interest. Remove a piece of the multilayered nonwoven web. For convenience of handing, the sample size may be a 75 mm by 75 mm piece, however, as will be appreciated by the person skilled in the art, smaller samples sizes can be used. If the multilayered nonwoven web needs to be removed from a product, such as an absorbent article, it may be necessary to use a cryogenic freeze spray (e.g. CytoFreeze, Control Company, TX) to remove the specimen from the product. Precondition samples at about 23°C $\pm$ 2 C° and about 50% $\pm$ 2% relative humidity for 2 hours prior to testing.

Place the specimen on the flat bed scanner, such that the first outer surface facing the glass, with the ruler directly adjacent. Placement is such that one of the side edges of the nonwoven web sample is parallel to the ruler. A black backing is placed over the specimen and the lid to the scanner is closed. Acquire an image composed of the nonwoven web and ruler at 4800 dpi in reflectance mode in 8 bit grayscale and save the file. Open the image file in ImageJ and perform a linear calibration using the imaged ruler.

Average Individual Bond Area

Enlarge a region of interest such that edges of the bonded areacan be clearly determined. With the area tool, manually trace the perimeter of a bonded area. Calculate and record the area to the nearest 0.001 mm$^2$. Repeat for a total of ten non-adjacent bonded areas randomly selected across the total specimen. A total of three substantially identical multilayered nonwoven web samples are measured for each sample set. Calculate the average and standard deviation of all 30 bond area.

If the bond pattern is such that the individual bonded areas are very different in size, the largest and the smallest bonded areas can each be determined as set out in the previous paragraph to determine the size range of the individual bonded areas.

Bond Area Percentage

Identify a single repeat pattern of bonded areas and unbonded areas and enlarge the image such that the repeat pattern fills the field of view. In ImageJ draw a box that encompasses the repeat pattern. If the bond pattern does not comprise a repeat pattern of bonded areas, a number of different samples is taken and measured such that the bond area percentage can be determined as an average value from these samples to a satisfying extend. The numbers of samples necessary may depend on the how unhomogeneous the bond pattern is (the number of samples may be from 10 to 1 00 or even more). Calculate area of the box and record to the nearest 0.01 mm$^2$. Next, with the area tool, trace the individual bonded area or portions thereof entirely within the box and calculate the areas of all bonded areas or portions thereof that are within the box. Record to the nearest 0.01 mm$^2$. Calculate as follows:

$$\text{Percent Bond Area} = (\text{Sum of areas of bond impressions within box}) / (\text{area of box}) \times 100\%$$

Repeat for a total of five non-adjacent regions of interest' s randomly selected across the total specimen.Record as Percent Bond Area to the nearest 0.01%. Calculate the average and standard deviation of all of the percent bond area measurements and report to the nearest 0.001 units.

If the bond pattern consists of continuous bonded areas with individual unbonded areas dispersed therein, basically the same test method can be used, however, rather than tracing the individual bonded areas, the unbonded areas are traced and the calculation is adjusted accordingly.

Opacity Measurement Method

The opacity of a material is the degree to which light is blocked by that material. A higher opacity value indicates a higher degree of light block by the material. Opacity may be measured using a 0.deg. illumination / 45.deg. detection, circumferential optical geometry, spectrophotometer with a computer interface such as the HunterLab LabScan XE running Universal Software (available from Hunter Associates Laboratory Inc., Reston, VA). Instrument calibration and measurements are made using the standard white and black calibration plates provided by the vendor. All testing is performed in a room maintained at about 23°C $\pm$ 2°C and about 50% $\pm$ 2% relative humidity. Configure the spectrophotometer for the XYZ color scale, D65 illuminant, 10.deg. standard observer, with UV filter set to nominal. Standardize the instrument according to the manufacturer's procedures using the 1.20 inch port size and 1.00 inch area view. After calibration, set the software to the Y opacity procedure.

To obtain the specimen, the multilayered nonwoven web has to be manufactured with only the layers lying above the second layer when viewed from the first outer surface. The resulting layered fabric consists thus either only of the first layer or, if the multilayered nonwoven web has additional layers between the first and second layer, consists of the first layer and these additional layers. The layer(s) is (are) pattern bonded the same way as the complete multilayered nonwoven web would have been bonded. Cut a piece 50.8 mm by 50.8 mm centered at each site identified above. Precondition samples at about 23°C $\pm$ 2°C and about 50% $\pm$ 2% relative humidity for 2 hours prior to testing.

Place the specimen over the measurement port. The specimen should completely cover the port with the first outer surface directed toward the port. Cover the specimen with the white standard plate. Take a reading, then remove the white tile and replace it with black standard tile without moving the specimen. Obtain a second reading, and calculate the opacity as follows:

$$\text{Opacity} = Y \text{ value [(black backing)} / Y \text{ value[(white backing) x 100}$$

A total of five substantially identical samples are analyzed and their opacity results recorded. Calculate and report the average opacity and standard deviation for the web measurements to the nearest 0.01%.Colorfastness Measurement

Colorfastness of the multilayered nonwoven web is measured following test method AATCC (American Association of Textile Chemists and Colorists) 116-2005 titled "Colorfastness to Crocking: Rotary Vertical Crockmeter Method", With regard to item 1.2 of the test method, colorfastness is measured taking a dry sample. The size of the test specimen may be smaller than 1 inch$^2$, which is indicated in item 7 of the test method. E.g. the sample may be as small as 25 mm long and 10 mm wide.

**Claims**

1. A multilayered nonwoven web comprising at least a first and a second layer, the first layer forming the first outer surface of the multilayered nonwoven web,
   wherein the multilayered nonwoven web is a nonwoven web which is made of several fiber layers, wherein the layers have been laid down, on top of one another, in one continuous manufacturing process, wherein the fibers of the multilayered nonwoven web are consolidated and bonded together to form a self-sustaining web only after the several layers of fibers have been laid down, such that the fibers within the different layers have not been substantially bonded together prior to assembling into a multilayered nonwoven web, but instead the fibers of all layers are pattern-bonded together after assembly into a multilayered nonwoven web,wherein the fibers of the first layer and the fibers of the second layer differ from each other in color,
   the multilayered nonwoven web being pattern bonded, wherein the bonded areas on the first outer surface have a first color and the unbonded areas on the first outer surface have a second color which is different from the first color, and wherein on the first outer surface, the delta E*, measured according to the test method set out in the description, between the bonded areas and the unbonded areas is at least 0.7.

2. The multilayered nonwoven web of claim 1, wherein the fibers of the first layer and the fibers of the second layer differ from each other in pigmentation.

3. The multilayered nonwoven web according to claim 1 or 2, wherein the fibers of the first layer and the fibers of the second layer are fused together in the bonded areas.

4. The multilayered nonwoven web according to any of the preceding claims, wherein the multilayered nonwoven web comprises one or more additional layer(s) positioned between the first layer and the second layer.

5. The multilayered nonwoven web according to claim 4, wherein at least one of the one or more additional layer(s) positioned between the first layer and the second layer is made of meltblown fibers or nanofibers and wherein the first layer is made of spunlaid fibers.

6. The multilayered nonwoven web according to any of the preceding claims, wherein the combined basis weight of the first layer and the optional additional layer(s) positioned between the first layer and the second layer is at least 1 gsm.

7. The multilayered nonwoven web according to any of the preceding claims, wherein the multilayered nonwoven web comprises one or more additional layer(s) positioned below the second layer when viewed from the first outer surface.

8. The multilayered nonwoven web according to any of the preceding claims, wherein the fibers of the first layer are made of fibers which are selected from the group consisting of shaped fibers, hollow fibers, nanofibers, fibers comprising at least 0.5 percent by weight of a white pigment, and combinations thereof.

9. The multilayered nonwoven web according to any of the preceding claims, wherein the first layer and the optional one or more additional layers positioned between the first layer and the second layer together have an opacity greater than 40%.

10. The multilayered nonwoven web according to any of the preceding claims, wherein the fibers of the second layer comprise a non- white pigment.

11. A disposable absorbent garment comprising the multilayered nonwoven web according to any of the preceding claims.

12. The disposable absorbent garment according to claim 11, wherein the disposable absorbent garment is selected from the group consisting of a diaper, a pant, a sanitary napkin and an absorbent insert for a diaper or pant, wherein the multilayered nonwoven web forms the topsheet, the backsheet, the back ears, the front ears, the landing zone, the elasticized leg cuffs and/or the barrier leg cuffs of the disposable absorbent article.

13. A disposable cleaning article comprising the multilayered nonwoven web according to any of claims 1 to 10.

14. Method of making a multilayered nonwoven web, wherein the multiiayered nonwoven web is a nonwoven web which is made of several fiber layers, wherein the layers have been laid down, on top of one another, in one continuous manufacturing process, wherein the fibers of the multiiayered nonwoven web are consolidated and bonded together to form a self-sustaining web only after the several layers of fibers have been laid down, such that the fibers within the different layers have not been substantially bonded together prior to assembling into a multilayered nonwoven web, but instead the fibers of all layers are pattern-bonded together after assembly into a multilayered nonwoven web, the method comprising the steps of

- laying down a first fibrous layer; and
- laying down a second fibrous layer, wherein the fibers of the first layer and fibers of the second layer differ from each other in color;
- bonding the first and second layers to each other by a bond pattern,

wherein the bonded areas on the first surface have a first color and the unbonded areas on the first surface have a second color, which is different from the first color and wherein on the first outer surface, the delta E*, measured according to the test method set out in the description, between the bonded areas and the unbonded areas is at least 1.0.

15. The method of claim 14, wherein the fibers of the first layer and the fibers of the second layer differ from each other in pigmentation.

16. The method of claim 14 or 15, wherein the fibers of the first and second layer are thermoplastic fibers.

17. The method of any of claims 14 to 16, wherein the bond pattern is imparted by the application of heat, pressure, ultrasonic energy or combinations thereof.

**Patentansprüche**

1. Mehrschichtiges Vlies, umfassend wenigstens eine erste und eine zweite Schicht, wobei die erste Schicht die erste Außenoberfläche des mehrschichtigen Vlieses bildet,
wobei das mehrschichtige Vlies ein Vlies ist, das aus mehreren Faserschichten besteht, wobei die Schichten in einem ununterbrochenem Herstellungsverfahren übereinander abgelegt worden sind, wobei die Fasern des mehrschichtigen Vlieses erst nach dem Ablegen der mehreren Faserschichten verfestigt und zu einem selbsttragenden Gewebe miteinander gebunden werden, so dass die Fasern innerhalb der verschiedenen Schichten vor dem Zusammenfügen zu einem mehrschichtigen Vlies nicht im Wesentlichen miteinander gebunden wurden, sondern stattdessen die Fasern aller Schichten nach dem Zusammenfügen zu einem mehrschichtigen Vlies mustergebunden werden, wobei sich die Fasern der ersten Schicht und die Fasern der zweiten Schicht in der Farbe voneinander unterscheiden,

wobei das mehrschichtige Vlies mustergebunden ist, wobei die gebundenen Bereiche auf der ersten Außenoberfläche eine erste Farbe aufweisen und die ungebundenen Bereiche auf der ersten Außenoberfläche eine zweite Farbe aufweisen, die sich von der ersten Farbe unterscheidet,

und wobei auf der ersten Außenoberfläche das Delta E*, gemessen nach dem in der Beschreibung dargelegten Prüfverfahren, zwischen den gebundenen Bereichen und den ungebundenen Bereichen wenigstens 0,7 beträgt.

2. Mehrschichtiges Vlies nach Anspruch 1, wobei sich die Fasern der ersten Schicht und die Fasern der zweiten Schicht in der Pigmentierung voneinander unterscheiden.

3. Mehrschichtiges Vlies nach Anspruch 1 oder 2, wobei die Fasern der ersten Schicht und die Fasern der zweiten Schicht in den gebundenen Bereichen miteinander verschmolzen sind.

4. Mehrschichtiges Vlies nach einem der vorstehenden Ansprüche, wobei das mehrschichtige Vlies eine oder mehrere zusätzliche Schicht(en) umfasst, die zwischen der ersten Schicht und der zweiten Schicht angeordnet ist/sind.

5. Mehrschichtiges Vlies nach Anspruch 4, wobei wenigstens eine der einen oder mehreren zusätzlichen Schicht(en), die zwischen der ersten Schicht und der zweiten Schicht angeordnet ist/sind, aus schmelzgeblasenen Fasern oder Nanofasern hergestellt ist und wobei die erste Schicht aus Spinnvlies hergestellt ist.

6. Mehrschichtiges Vlies nach einem der vorstehenden Ansprüche, wobei das kombinierte Flächengewicht der ersten Schicht und der optionalen zusätzlichen Schicht(en), die zwischen der ersten Schicht und der zweiten Schicht angeordnet ist/sind, wenigstens 1 Gramm pro Quadratmeter beträgt.

7. Mehrschichtiges Vlies nach einem der vorstehenden Ansprüche, wobei das mehrschichtige Vlies eine oder mehrere zusätzliche Schicht(en) umfasst, die bei Betrachtung von der ersten Außenoberfläche unter der zweiten Schicht angeordnet ist/sind.

8. Mehrschichtiges Vlies nach einem der vorstehenden Ansprüche, wobei die Fasern der ersten Schicht aus Fasern hergestellt sind, die ausgewählt sind aus der Gruppe bestehend aus geformten Fasern, Hohlfasern, Nanofasern, Fasern, die wenigstens 0,5 Gewichtsprozent eines weißen Pigments umfassen, und Kombinationen davon.

9. Mehrschichtiges Vlies nach einem der vorstehenden Ansprüche, wobei die erste Schicht und die optional eine oder mehreren zusätzlichen Schichten, die zwischen der ersten Schicht und der zweiten Schicht angeordnet sind, zusammen eine Trübung von mehr als 40 % aufweisen.

10. Mehrschichtiges Vlies nach einem der vorstehenden Ansprüche, wobei die Fasern der zweiten Schicht ein nicht weißes Pigment umfassen.

11. Einweg-Absorptionskleidungsstück, umfassend das mehrschichtige Vlies nach einem der vorstehenden Ansprüche.

12. Einweg-Absorptionskleidungsstück nach Anspruch 11, wobei das Einweg-Absorptionskleidungsstück ausgewählt ist aus der Gruppe bestehend aus einer Windel, einem Höschen, einer Damenbinde und einem Absorptionseinsatz für eine Windel oder ein Höschen, wobei das mehrschichtige Vlies die Oberschicht, die Unterschicht, die rückseitigen Seitenlappen, die vorderseitigen Seitenlappen, die Auftreffzone, die elastifizierten Beinbündchen und/oder die Sperrbeinbündchen des Einweg-Absorptionsartikels bildet.

13. Einweg-Reinigungsartikel, umfassend das mehrschichtige Vlies nach einem der Ansprüche 1 bis 10.

14. Verfahren zum Herstellen eines mehrschichtigen Vlieses, wobei das mehrschichtige Vlies ein Vlies ist, das aus mehreren Faserschichten besteht, wobei die Schichten in einem ununterbrochenem Herstellungsverfahren übereinander abgelegt worden sind, wobei die Fasern des mehrschichtigen Vlieses erst nach dem Ablegen der mehreren Faserschichten verfestigt und zu einem selbsttragenden Gewebe miteinander gebunden werden, so dass die Fasern innerhalb der verschiedenen Schichten vor dem Zusammenfügen zu einem mehrschichtigen Vlies nicht im Wesentlichen miteinander gebunden wurden, sondern stattdessen die Fasern aller Schichten nach dem Zusammenfügen zu einem mehrschichtigen Vlies mustergebunden werden, wobei das Verfahren die folgenden Schritte umfasst

- Ablegen einer ersten Faserschicht; und
- Ablegen einer zweiten Faserschicht, wobei sich die Fasern der ersten Schicht und die Fasern der zweiten Schicht in der Farbe voneinander unterscheiden;
- Verbinden der ersten und der zweiten Schicht miteinander durch ein Verbindungsmuster,

wobei die gebundenen Bereiche auf der ersten Oberfläche eine erste Farbe aufweisen und die ungebundenen Bereiche auf der ersten Oberfläche eine zweite Farbe aufweisen, die sich von der ersten Farbe unterscheidet, und wobei auf der ersten Außenoberfläche das Delta E*, gemessen nach dem in der Beschreibung dargelegten Prüfverfahren, zwischen den gebundenen Bereichen und den ungebundenen Bereichen wenigstens 1,0 beträgt.

**15.** Verfahren nach Anspruch 14, wobei sich die Fasern der ersten Schicht und die Fasern der zweiten Schicht in der Pigmentierung voneinander unterscheiden.

**16.** Verfahren nach Anspruch 14 oder 15, wobei die Fasern der ersten und zweiten Schicht Thermoplastfasern sind.

**17.** Verfahren nach einem der Ansprüche 14 bis 16, wobei das Verbindungsmuster durch die Anwendung von Wärme, Druck, Ultraschallenergie oder Kombinationen davon verliehen wird.

## Revendications

**1.** Nappe non tissée multicouche comprenant au moins des première et deuxième couches, la première couche formant la première surface externe de la nappe non tissée multicouche,
dans lequel la nappe non tissée multicouche est une nappe non tissée qui est constituée de plusieurs couches fibreuses, dans laquelle les couches ont été déposées, les unes sur les autres, dans un procédé de fabrication continue, dans laquelle les fibres de la nappe non tissée multicouche sont consolidées et liées ensemble pour former une nappe autoporteuse uniquement après que les différentes couches de fibres ont été déposées, de telle sorte que les fibres dans les différentes couches n'ont pas été essentiellement liées ensemble avant assemblage en une nappe non tissée multicouche, mais à la place les fibres de toutes les couches sont liées par un motif ensemble après assemblage en une nappe non tissée multicouche, dans laquelle les fibres de la première couche et les fibres de la deuxième couche diffèrent l'une de l'autre en couleur,
la nappe non tissée multicouche étant liée par motif, dans laquelle les zones liées sur la première surface externe présentent une première couleur et les zones non liées sur la première surface externe présentent une deuxième couleur qui est différente de la première couleur,
et dans laquelle, sur la première surface extérieure, le delta E*, mesuré selon le procédé de test mis en œuvre dans la description, entre les zones liées et les zones non liées est d'au moins 0,7.

**2.** Nappe non tissée multicouche selon la revendication 1, dans laquelle les fibres de la première couche et les fibres de la deuxième couche diffèrent l'une de l'autre dans la pigmentation.

**3.** Nappe non tissée multicouche selon la revendication 1 ou 2, dans laquelle les fibres de la première couche et les fibres de la deuxième couche sont fusionnées ensemble dans les zones liées.

**4.** Nappe non tissée multicouche selon l'une quelconque des revendications précédentes, dans laquelle la nappe non tissée multicouche comprend une ou plusieurs couche(s) supplémentaire(s) positionnée(s) entre la première couche et la deuxième couche.

**5.** Nappe non tissée multicouche selon la revendication 4, dans laquelle au moins l'une de la ou des couche(s) supplémentaire(s) positionnée(s) entre la première couche et la deuxième couche est constituée de fibres ou nanofibres soufflées par fusion et dans laquelle la première couche est constituée de fibres obtenues par voie fondue.

**6.** Nappe non tissée multicouche selon l'une quelconque des revendications précédentes, dans laquelle la masse surfacique combinée de la première couche et de la ou des couche(s) supplémentaire(s) facultative(s) positionnée(s) entre la première couche et la deuxième couche est d'au moins 1 g/m$^2$.

7. Nappe non tissée multicouche selon l'une quelconque des revendications précédentes, dans laquelle la nappe non tissée multicouche comprend une ou plusieurs couche(s) supplémentaire(s) positionnée(s) sous la deuxième couche lorsqu'elles sont vues à partir de la première surface extérieure.

8. Nappe non tissée multicouche selon l'une quelconque des revendications précédentes, dans laquelle les fibres de la première couche sont constituées de fibres qui sont choisies dans le groupe constitué des fibres façonnées, des fibres creuses, des nanofibres, des fibres comprenant au moins 0,5 pour cent en poids d'un pigment blanc, et leurs combinaisons.

9. Nappe non tissée multicouche selon l'une quelconque des revendications précédentes, dans laquelle la première couche et la ou les couche(s) supplémentaire(s) facultative(s) positionnée(s) entre la première couche et la deuxième couche présentent ensemble une opacité supérieure à 40 %.

10. Nappe non tissée multicouche selon l'une quelconque des revendications précédentes, dans laquelle les fibres de la deuxième couche comprennent un pigment non blanc.

11. Vêtement absorbant jetable comprenant la nappe non tissée multicouche selon l'une quelconque des revendications précédentes.

12. Vêtement absorbant jetable selon la revendication 11, dans lequel le vêtement absorbant jetable est choisi dans le groupe constitué d'une couche, d'une culotte, d'une serviette hygiénique et d'un insert absorbant pour une couche ou une culotte, dans lequel la nappe non tissée multicouche forme la feuille de dessus, la feuille de support, les languettes arrière, les languettes avant, la zone de réception, les revers de jambe élasticisés et/ou les revers de jambe formant barrière de l'article absorbant jetable.

13. Article de nettoyage jetable comprenant la nappe non tissée multicouche selon l'une quelconque des revendications 1 à 10.

14. Procédé de fabrication d'une nappe non tissée multicouche, dans lequel la nappe non tissée multicouche est une nappe non tissée qui est constituée de plusieurs couches fibreuses, dans lequel les couches ont été disposées, les unes sur les autres, dans un procédé de fabrication continue, dans lequel les fibres de la nappe non tissée multicouche sont consolidées et liées ensemble pour former une nappe autoporteuse uniquement lorsque les différentes couches de fibres ont été déposées, de telle sorte que les fibres dans les différentes couches n'ont pas été essentiellement liées ensemble avant assemblage en une nappe non tissée multicouche, mais à la place les fibres de toutes les couches sont liées par motif ensemble après assemblage en une nappe non tissée multicouche, le procédé comprenant les étapes consistant à

   - déposer une première couche fibreuse ; et
   - déposer une deuxième couche fibreuse, dans lequel les fibres de la première couche et les fibres de la deuxième couche diffèrent l'une de l'autre en couleur ;
   - lier les première et deuxième couches l'une à l'autre par une liaison par motif,

dans lequel les zones liées sur la première surface ont une première couleur et les zones non liées sur la première surface ont une deuxième couleur différente de la première couleur et dans lequel, sur la première surface externe, le delta E* mesuré selon le procédé de test mis en œuvre dans la description, entre les zones liées et les zones non liées, est d'au moins 1,0.

15. Procédé selon la revendication 14, dans lequel les fibres de la première couche et les fibres de la deuxième couche diffèrent l'une de l'autre dans la pigmentation.

16. Procédé selon la revendication 14 ou 15, dans lequel les fibres des première et deuxième couches sont des fibres thermoplastiques.

17. Procédé selon l'une quelconque des revendications 14 à 16, dans lequel le motif de liaison est réalisé par l'application de chaleur, de pression, d'énergie ultrasonique ou de combinaisons de celles-ci.

# Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006056892 A
- US 6557224 B
- US 3860003 A
- US 5221274 A
- US 5554145 A
- US 5569234 A
- US 5580411 A
- US 6004306 A
- US 7922943 B2
- US 7931457 B2
- US 6616435 B2
- US 5266392 A
- US 5370764 A